# FASCICULE DE BREVET EUROPEEN

(11) **EP 4 271 522 B1**
(45) Date de publication et mention de la délivrance du brevet: **27.05.2026**
(21) Numéro de dépôt: 23700805.7
(22) Date de dépôt: 12.01.2023
(51) Int. Cl.: B01L 3/00

(54) **DISPOSITIF MICROFLUIDIQUE D'OBTENTION D'UN MÉLANGE PLASMA-RÉACTIF ET PROCÉDÉ DE MISE EN OEUVRE**
MIKROFLUIDISCHE VORRICHTUNG ZUR HERSTELLUNG EINER PLASMA-REAGENZ-MISCHUNG UND VERFAHREN ZUR IMPLEMENTIERUNG DAVON
MICROFLUIDIC DEVICE FOR OBTAINING A PLASMA-REAGENT MIXTURE, AND METHOD FOR IMPLEMENTING SAME

(30) Priorité: 25.03.2022 FR 2202677
(43) Date de publication de la demande: 08.11.2023
(73) Titulaire: Belmont Diagnostics, 13510 Eguilles (FR)
(72) Inventeur: KALISZCZAK, Maciej, ST BELMONT, Massachusetts 02478 (US); VIGNERAS, Aymeric, BELMONT, Massachusetts 02478 (US); VIGNERAS, Erwan, 29000 Quimper (FR)
(74) Mandataire: Brun, Philippe Alexandre Georges
(86) Numéro de dépôt international: PCT/EP2023/050672
(87) Numéro de publication internationale: WO 2023/179927

(56) Documents cités:
- WO-A1-2014/172247
- US-A1- 2012 275 955
- US-A1- 2014 309 557

## Description

La présente invention a trait, de manière générale, au domaine des dispositifs de test sanguin. L'invention concerne plus particulièrement un dispositif microfluidique d'obtention d'un mélange plasma-réactif et un procédé de mise en œuvre d'un tel dispositif afin de permettre, *in fine*, la quantification d'un biomarqueur sanguin d'intérêt.

En 2019, d'après l'Organisation Mondiale de la Santé, sept des dix principales causes de décès à l'échelle mondiale étaient des maladies chroniques et le pourcentage de décès liés à de telles maladies est en constante augmentation. Les maladies chroniques sont définies comme des maladies évolutives présentant une ancienneté de plusieurs mois. Or, de nombreuses études ont montré qu'une détection précoce de telles maladies augmente les chances de survie d'un patient. Pour exemple, dans le cas de plusieurs types de cancer, la survie peut être plus de trois fois supérieure lorsque le cancer est diagnostiqué précocement (stade un ou deux). De même, une détection précoce de marqueurs liés à des maladies ou symptômes aigus ainsi que d'agents pathogènes, tels que, par exemple, le virus du Covid 19, l'élément pathogène associé à la maladie de Lyme ou celui lié à la septicémie ou encore le Virus de l'Immunodéficience Humaine (connu sous l'acronyme VIH), permet d'évaluer le risque de forme pathologique grave et d'orienter rapidement la prise en charge, pouvant nécessiter d'être immédiate et vitale dans certains cas, afin de favoriser une guérison.

Pour cela, les biomarqueurs sanguins, caractéristiques biologiques, suscitent un intérêt croissant comme indicateur d'un processus biologique normal ou pathologique. À ce titre, chaque biomarqueur dispose de caractéristiques propres qui lui permettent d'apporter des informations en lien avec un processus physiopathologique sous-jacent et/ou des données de progression d'une maladie. Par analogie, les biomarqueurs sont aux médecins ce que les empreintes digitales sont aux policiers : de véritables signatures qui permettent d'identifier non pas un individu mais une maladie. Les biomarqueurs consistent en des molécules (protéines, hormones, etc.) et des cellules dont la présence ou la concentration anormale dans le sang, plus particulièrement dans le plasma sanguin, atteste de l'existence d'une pathologie. Le plasma sanguin est le composant liquide du sang dépourvu des globules rouges, des globules blancs, des plaquettes et autres contaminants, constituant environ cinquante-cinq pourcents du volume total du sang et contiendrait au moins trois cents protéines.

Un biomarqueur peut être présent chez les patients atteints d'une maladie précise et absent chez les individus en bonne santé ou touchés par d'autres maladies. Cependant, une quantification précise d'un biomarqueur permet d'être le reflet de l'évolution d'une pathologie. Pour exemple, la quantité d'un biomarqueur peut augmenter ou diminuer selon que la maladie s'aggrave ou s'améliore, et inversement. Ainsi, de tels indicateurs quantitatifs permettent ;
- d'affiner un pronostic en délivrant une information complète pour caractériser une maladie (ou la présence d'un agent pathogène) chez un individu et en optimiser sa prise en charge ;
- d'adapter à un patient le traitement qui lui convient le mieux. A ce titre, en fonction de leurs métabolismes, certaines personnes assimilent particulièrement rapidement un médicament. Ce dernier peut passer, dans ce cas, plus rapidement dans le sang, ses effets sont accrus et il est donc nécessaire de diminuer les doses habituellement prescrites ;
- de suivre les effets d'une thérapie. Pour exemple, dans le cas de cancer, les cellules cancéreuses libèrent diverses molécules dans la circulation sanguine. Si leur concentration augmente au cours du temps, cela peut signifier que le cancer récidive. La quantification des biomarqueurs sanguins apparait donc comme un outil indispensable à une médecine personnalisée.

Traditionnellement, une telle quantification est possible en ayant recours au processus conventionnel et éprouvé de la prise de sang. Cependant, un tel processus nécessite une quantité importante de sang, l'intervention d'un personnel qualifié et l'utilisation d'équipement spécialisé, ce qui procure une technique coûteuse et chronophage. En effet, cela demande de disposer d'une prescription d'un médecin pour requérir une prise de sang, de prendre un rendez-vous auprès d'un infirmier ou d'un laboratoire supposant une disponibilité commune du personnel de santé et du patient, d'attendre la réalisation des tests sanguins en laboratoire et la transmission des résultats au médecin suivie de l'interprétation de ces derniers.

Pour pallier une partie des étapes chronophages précitées, il est également connu, sur le marché, des kits d'auto-prélèvement sanguin pouvant être réalisés à domicile par tout individu, utilisant un volume de sang moins important, tel que le divulgue les demandes de brevet US 2019/0111421, US2014/0309557 ou WO2014/172247A1 en proposant des dispositifs de collecte et de stockage ou transfert d'un fluide corporel, en l'espèce du sang. Toutefois, comme dans le cas de la prise de sang, de tels dispositifs nécessitent les services d'un laboratoire pour la réalisation des tests avec un équipement spécialisé et l'analyse des résultats par un personnel qualifié.

La demande de brevet US 2017/0001192 tente de répondre en partie à ces inconvénients en proposant un dispositif de test permettant l'évaluation de biomarqueurs dans un fluide corporel sans avoir recours à une ou des ressources extérieures (laboratoire, personnel qualifié, ...). Cependant, un tel dispositif permet d'aboutir à un résultat qualitatif d'un biomarqueur (présence ou non du biomarqueur) mais non à un résultat quantitatif de tout biomarqueur. Une telle solution requiert ainsi une analyse en laboratoire pour quantifier le biomarqueur d'intérêt et ne permet pas d'identifier des protéines en faible abondance (de l'ordre de microgramme-nanogramme/millilitre et moins) pouvant être des marqueurs essentiels.

Les documents US2014/0309557 et WO2014/172247A1 décrivent un dispositif de prélèvement, de stockage et de transfert d'un échantillon de sang. Un tel dispositif comporte, préalablement audit prélèvement sanguin, un additif anticoagulant pour assurer un acheminement dudit échantillon de sang vers un laboratoire en chargé de réaliser un test portant sur du plasma tiré dudit échantillon de sang. Toutefois, à l'instar d'un processus conventionnel de prise de sang, la séparation du plasma des autres constituants du sang nécessite l'emploi d'équipements spécifiques en laboratoire, en l'espèce notamment une centrifugeuse, et un personnel qualifié.

Seul un résultat quantitatif de biomarqueurs permet, comme précédemment indiqué, une surveillance renforcée de la santé d'un patient. Ainsi, la présente invention vise à remédier aux inconvénients précités, notamment à proposer un dispositif microfluidique d'obtention d'un mélange homogène plasma-réactif et son procédé de mise en œuvre simplifié afin de disposer, *in fine*, d'une quantification directe et rapide d'un biomarqueur sanguin, à domicile, exploitable à partir d'une seule goutte de sang et pouvant être mis en œuvre par tout utilisateur tout en assurant une fiabilité et reproductibilité des résultats du fait d'un dosage précis/d'un contrôle des volumes, à toutes les étapes du procédé, du plasma sanguin et du réactif utilisé.

À ce titre, un premier objet de l'invention concerne un dispositif microfluidique d'obtention d'un mélange homogène plasma-réactif comprenant :
- un module de collecte de sang ;
- un premier canal capillaire présentant une première extrémité en connexion fluidique avec ledit module de collecte ;
- un réservoir de stockage d'un fluide en connexion fluidique avec ledit premier canal capillaire ;
- une chambre de mélange en connexion fluidique avec une deuxième extrémité du premier canal capillaire agencée pour provoquer passivement un mélange homogène de fluides issus dudit premier capillaire et présentant une sortie d'écoulement dudit mélange homogène ;
- un moyen de prévention de tout retour fluidique vers le module de collecte.

Pour réaliser un test à domicile ou en mobilité et ainsi prévenir la nécessité d'utiliser des équipements tiers et d'effectuer un test en laboratoire par un personnel spécialisé, un tel dispositif microfluidique d'obtention d'un mélange homogène plasma-réactif est agencé de sorte que le module de collecte de plasma comporte :
- une membrane semi-perméable agencée pour recevoir une quantité de sang et conçue pour séparer, par gravité lorsque ladite membrane semi-perméable est sensiblement horizontale, les constituants du sang selon leurs tailles et ainsi piéger lesdits constituants autres que le plasma sanguin ;
- une surface spécifique agencée entre ladite membrane semi-perméable et la première extrémité du premier canal capillaire et présentant des propriétés hydrophobes, hydrophiles et de tension de surface pour extraire le plasma sanguin de la membrane semi-perméable et faire circuler ledit plasma sanguin dans ledit premier canal capillaire.

En outre, un tel dispositif microfluidique d'obtention d'un mélange homogène plasma-réactif est agencé de sorte que :
- le premier canal capillaire présente des dimensions déterminées entre la connexion fluidique dudit réservoir de stockage avec le premier canal capillaire et ladite deuxième extrémité dudit premier canal capillaire, pour maîtriser la quantité de plasma sanguin lorsque le premier canal capillaire est saturé en plasma sanguin ;
- le fluide contenu dans le réservoir de stockage est un réactif destiné à être mélangé avec du plasma sanguin ;
- ledit réservoir de stockage :
   ∘ est adapté à la réception d'un volume de réactif supérieur à la somme des volumes de fluides que peuvent contenir le premier canal capillaire et la chambre de mélange ;
   ∘ comporte un actionneur conçu pour provoquer un écoulement forcé dudit réactif dans le premier canal capillaire entraînant un écoulement d'un mélange homogène de plasma-réactif par ladite sortie s'écoulement de la chambre de mélange lorsque ledit actionneur est commandé après saturation du premier canal capillaire en plasma sanguin.

Afin d'optimiser le phénomène de capillarité et de maximiser la mise en mouvement du plasma sanguin depuis la membrane semi-perméable vers le premier canal capillaire la première extrémité dudit premier canal capillaire peut être est positionnée sensiblement au centre dudit module de collecte.

Pour concentrer le sang déposé sur la membrane semi-perméable et toute propagation du sang en dehors de la surface de celle-ci, le module de collecte d'un tel dispositif peut comporter un porte-membrane agencé pour concentrer le sang sur ladite membrane semi-perméable.

A titre avantageux, la surface spécifique peut être constituée de polyméthacrylate de méthyle, polymère thermoplastique, de dérivés de polyacrylamide, de polyuréthane, de poly[hydroxyéthyl méthacrylamide] ou de poly[éthylène glycol].

Pour optimiser un mélange passif et homogène du plasma sanguin et du réactif circulant dans ledit premier canal capillaire, ladite chambre de mélange peut comporter un réseau de canaux respectivement agencés en zigzags, serpentins ou en chevrons pour provoquer passivement ledit mélange homogène de plasma-réactif.

Un deuxième objet de l'invention consiste en un procédé de mise en œuvre d'un dispositif microfluidique d'obtention d'un mélange homogène plasma-réactif conforme à l'invention. Un tel procédé comporte les étapes suivantes :
- une étape de dépôt de sang sur la membrane semi-perméable du module de collecte de plasma, lorsque ledit dispositif est positionné de sorte que la membrane semi-perméable est horizontale ;
- une étape de saturation du premier canal capillaire en plasma sanguin ;
- une étape, postérieure à ladite étape de saturation du premier canal capillaire en plasma sanguin, de commande de l'actionneur du réservoir de stockage provoquant l'écoulement forcé d'un réactif, préalablement contenu dans ledit réservoir de stockage, dans le premier canal capillaire au-delà d'une saturation de la chambre de mélange ;
- une étape de collecte d'un mélange homogène plasma-réactif depuis la sortie d'écoulement de cette dernière.

A titre d'exemple de mise en œuvre avantageux, l'étape de saturation du premier canal capillaire en plasma sanguin peut consister à attendre, dès lors que le plasma sanguin s'écoule dans le premier canal capillaire, une durée prédéterminée, fonction du dimensionnement du premier canal capillaire.

Pour réaliser une analyse dudit mélange plasma-réactif, l'étape de collecte du mélange homogène plasma-réactif peut consister à positionner une bandelette réactive en aval de la sortie d'écoulement de la chambre de mélange de sorte que ladite bandelette recueille tout ou partie dudit mélange homogène plasma-réactif.

L'exposé de l'invention sera maintenant poursuivi par la description détaillée d'un exemple de réalisation, donné ci-après à titre illustratif mais non limitatif, en référence aux dessins annexés, sur lesquels :
- La figure 1 montre un exemple préféré de mise en œuvre d'un dispositif microfluidique d'obtention d'un mélange plasma-réactif selon l'invention ;
- La figure 2 montre un organigramme représentatif d'étapes du procédé de mise en œuvre d'un dispositif conforme à l'invention ;
- Les figure 3 à 7 sont des vues schématiques du dispositif selon l'invention illustrant différentes étapes de mise en œuvre dudit dispositif de selon l'invention.

En préambule, il est important de rappeler la définition des termes suivants :
- l'hydrophilie est un terme utilisé pour caractériser une propriété moléculaire résultant de l'existence de fortes attractions ou affinités entre certains groupements moléculaires et l'eau. En d'autres termes, c'est l'affinité d'une substance pour l'eau ou l'aptitude à être mouillée par l'eau sans être dissoute ;
- l'hydrophobie est un terme que l'on utilise pour désigner et caractériser les surfaces qui semblent « repousser l'eau » ou que l'eau semble avoir du mal à mouiller, notamment du fait que les molécules d'eau soient polaires et aient une attirance préférentielle pour les autres molécules polaires ;
- la tension superficielle/l'énergie de surface et l'angle de contact : on associe à une interface qui sépare deux milieux différents, une énergie par unité de surface, dont l'origine est la force de cohésion entre molécules identiques. Un exemple représentatif d'une telle grandeur est celui d'une goutte de liquide déposée sur un support/solide : une telle goutte formant un angle, appelé angle de contact, qui varie en fonction de la tension superficielle du support/solide. Ainsi, s'il y a répulsion entre le liquide et le solide, la goutte posée sur le solide va avoir tendance à « se regrouper » et à prendre une forme sphérique, présentant ainsi un angle de contact « élevé ». Inversement, s'il y a attraction entre le liquide et le solide, la goutte posée sur le solide va avoir tendance à s'étaler, présentant ainsi un angle de contact « faible ». Dans le cas d'un liquide tel que l'eau, si l'angle de contact est supérieur à zéro degré et inférieur à quatre-vingt-dix degrés, l'eau tend à s'étaler et à mouiller le support/solide : on parle alors de surface mouillante ou hydrophile. Dans le cas où l'angle de contact est sensiblement égal à zéro degré, le mouillage est total. Ainsi, plus l'angle de contact est élevé, moins le support/solide est mouillable par l'eau ;
- la capillarité est une propriété des fluides dépendant de la tension superficielle de tels fluides, leur donnant la capacité de monter ou de descendre à travers un tube/canal capillaire. La distance parcourue par un liquide dans le tube est d'autant plus grande que ledit tube est fin ;
- le plasma sanguin est la partie liquide du sang composée à plus de quatre-vingt-dix pourcents d'eau. Ainsi, le plasma sanguin est considéré comme principalement hydrophile.

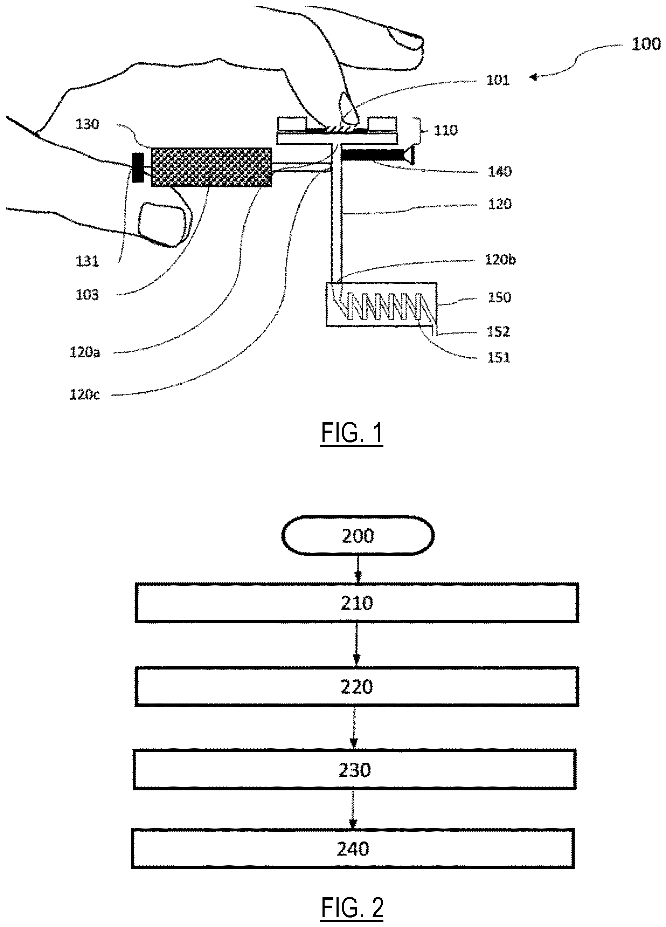

Un exemple préféré de dispositif 100 microfluidique d'obtention d'un mélange homogène plasma-réactif, conforme à l'invention, est représenté sur la figure 1. Ledit dispositif 100 est composé d'au moins un module de collecte 110 de plasma sanguin en connexion fluidique avec une première extrémité 120a d'un canal capillaire 120, lui-même en connexion fluidique avec un réservoir de stockage 130 d'un réactif 103. Au sens de l'invention, on entend par « réactif » tout fluide permettant de rendre certains paramètres physico-chimiques du plasma sanguin, tel que le pH par exemple, optimaux pour la conformation d'une protéine objet d'une quantification. Un tel réactif peut en outre être choisi pour contrôler la vitesse d'écoulement fluidique dans le dispositif et limiter toute interaction non spécifique des protéines d'intérêts (biomarqueurs) avec la membrane semi-perméable et les parois internes du dispositif. A titre d'exemples non limitatif, il peut être composé de protéines comme le BSA (« *Bovine Serum Albumin »* selon une terminologie anglo-saxonne), de détergent (tel que Tween 20), d'une solution saline (telle que le « *Phosphate Buffer Saline* » selon une terminologie anglo-saxonne) et/ou d'une solution antibactérienne (telle que « *Sodium Azide »* selon une terminologie anglo-saxonne). Un tel réactif se distingue de stabilisants ou anticoagulants que l'on trouve dans certains dispositifs de prélèvement et de transport de sang tels que décrits par exemple dans les documents US2014/0309557 et WO2014/172247A1 évoqués précédemment, dont la seule fonction est de permettre le stockage d'échantillons de sang jusqu'à un centre d'analyse et qui ne nécessitent pas d'être mis en mouvement au sein desdits dispositifs de transport. Ledit dispositif 100 comporte, en outre, un moyen 140 de prévention de tout retour fluidique vers ledit module de collecte 110, positionné en amont de la connexion fluidique dudit réservoir de stockage 130 avec ledit canal capillaire 120. Le dispositif 100 comporte également une chambre de mélange 150 en connexion fluidique avec une deuxième extrémité 120b du canal capillaire 120. Ledit dispositif 100 est mis en œuvre par ledit procédé 200, illustré sur l'organigramme en figure 2, afin d'obtenir un mélange 104 homogène de plasma-réactif à partir d'une quantité minimale de sang 101, plus particulièrement de plasma sanguin, de l'ordre de dix à cent microlitres, un tel mélange 104 permettant, *in fine*, la quantification d'un biomarqueur sanguin d'intérêt en utilisant une bandelette de test directement sur place, c'est-à-dire, par exemple, à domicile par la personne ayant effectué le prélèvement sanguin, soit généralement le patient, ou au chevet de ce dernier, en ambulance, voire au sein du cabinet de consultation d'un médecin.

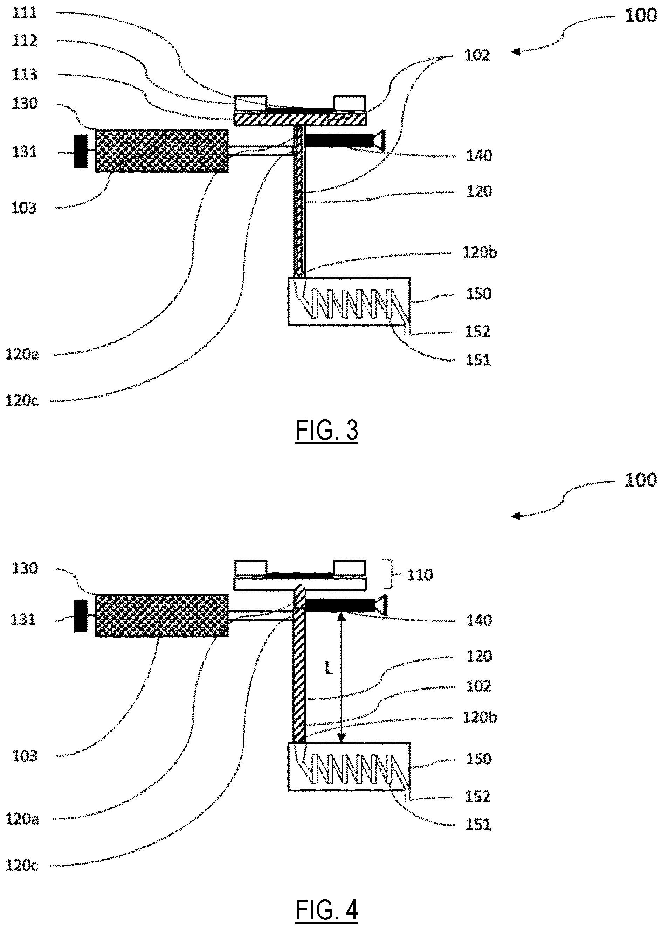

Une étape 210 dudit procédé 200 consiste à alimenter en plasma sanguin 102 ledit module de collecte 110 qui est en connexion fluidique avec la première extrémité 120a dudit canal capillaire 120, considérée comme la partie haute dudit canal capillaire 120, la deuxième extrémité 120b étant considérée comme la partie basse dudit canal capillaire 120 lorsque ledit canal capillaire 120 est orienté dans l'espace comme l'indique la figure 1. Une telle alimentation en plasma sanguin 102 peut se traduire par le dépôt de sang dans le module de collecte 110, lorsque ledit module de collecte 110 est agencé pour passivement séparer ledit plasma sanguin 102 dudit sang, ou consister en un dépôt direct de plasma dans ledit module de collecte. Ainsi, tel qu'illustré en figure 3, ledit plasma sanguin 102 peut se déplacer automatiquement dans le canal capillaire 120 par capillarité, phénomène défini précédemment dans la présente description et ainsi s'écouler verticalement (de haut en bas selon la figure 1) le long dudit canal 120. Dans un mode de réalisation préféré mais non limitatif, afin d'optimiser un tel phénomène de capillarité et de maximiser l'effet associé, il est préférable que la première extrémité 120a du canal capillaire 120 soit positionnée sensiblement au centre de la surface inférieure du module de collecte 110. En variante, ladite première extrémité 120a du canal capillaire 120 peut être positionnée entre le bord extérieur et le centre de la surface inférieure du module de collecte 110.

Comme évoqué précédemment, le plasma sanguin 102 peut être avantageusement isolé, à l'aide dudit dispositif 100 conforme à l'invention, à partir d'une ou plusieurs gouttes de sang 101 prélevées par l'utilisateur lui-même. Une telle goutte 101 peut être recueillie par exemple à l'aide d'un dispositif à lancette que l'utilisateur vient appliquer au bout de son doigt ou tout autre dispositif adapté. Dès lors que le bout du doigt de l'utilisateur est percé ou piqué par la lancette, l'utilisateur peut ainsi déplacer son doigt au niveau du module de collecte 110 de plasma sanguin pour y déposer la goutte de sang 101, tel qu'illustré en figure 1. À ce titre, tel qu'illustré en figure 3, dans un exemple préféré, ledit module 110 comporte une membrane semi-perméable 111 à travers laquelle le sang 101 collecté peut passer ou s'écouler. Une telle membrane semi-perméable 111, par exemple de marque Pall Vivid^{®} connue et disponible sur le marché, permet ainsi de séparer les constituants du sang en fonction de leurs tailles. Ladite membrane semi-perméable 111 est ainsi agencée pour recevoir une quantité de sang 101 et séparer, par gravité lorsque ladite membrane semi-perméable 111 est sensiblement horizontale, les constituants du sang selon leurs tailles de sorte que les globules blancs, les globules rouges et autres constituants de grand diamètre puissent être piégés dans ladite membrane 111 au contraire du plasma sanguin 102 pouvant s'écouler au travers de ladite membrane. Il est considéré un pourcentage d'efficacité de filtrage de l'ordre de soixante pourcents ou plus. Ainsi, par gravité, lorsque ladite membrane 111 est sensiblement horizontale, le plasma sanguin 102 peut être collecté en deçà de ladite membrane 111.

En outre, afin de concentrer le sang 101 sur ladite membrane 111 et d'éviter que la sang 101 ne se propage en dehors de la surface de séparation/filtration, délimitée par les bords de ladite membrane 111, le module de collecte 110 peut comporter un porte-membrane 112 positionné de manière à presser et maintenir les bords de ladite membrane 111. Toutefois, l'Homme du métier ne saurait se limiter à un tel dispositif de porte-membrane 112 et pourra envisager tout autre type de dispositif permettant de remplir cette fonction.

Une fois que le plasma sanguin 102 a pu être séparé des autres constituants du sang, il faut pouvoir le récupérer pour permettre la mise en continuité dudit plasma 102 dans ledit dispositif 100. À ce titre, ledit plasma sanguin 102 doit être retiré de la membrane semi-perméable 111 mais aussi acheminé jusqu'au canal capillaire 120. L'invention se distingue clairement de l'enseignement tiré du document US2012/0275955A1 qui impose la présence d'une chambre de dépression pour créer un flux et séparer de manière forcée du plasma d'un échantillon de sang au travers d'une membrane. Une telle filtration forcée est susceptible de déformer la membrane, voire d'engendrer un phénomène connu d'hémolyse selon lequel des globules rouges peuvent se briser sous l'effet dudit flux au travers de la membrane altérant ainsi le plasma obtenu. En effet, les constituants cellulaires libérés (e.g., potassium, lactate déshydrogénase, hémoglobine) lors d'une telle destruction peuvent altérer la pertinence et la précision d'une quantification subséquente de protéines à partir dudit plasma hémolysé. Contrairement à un tel enseignement technique tiré du document US2012/0275955A1, tel qu'illustré en figure 1, le module de collecte 110 selon l'invention peut comporter une surface spécifique 113, positionnée en amont de la connexion fluidique dudit module de collecte de plasma 110 et de la première extrémité 120a du canal capillaire 120. Une telle surface spécifique 113 permet, par gravité, d'extraire le plasma 102 de ladite membrane semi-perméable 111 et de le faire circuler et progresser par capillarité au sein du dispositif 100 jusque dans le canal capillaire 120. L'invention permet ainsi de prévenir tout risque d'hémolyse ou de déformation de la membrane semi-perméable 111 préservant le plasma sanguin 102 ainsi isolé et par voie de conséquence maintient la pertinence de toute quantification portant sur ledit plasma sanguin 102.

Pour permettre la mise en continuité du plasma sanguin 102 dans le dispositif 100 et pas seulement la rétention dudit plasma 102, en d'autres termes absorber le plasma sanguin 102 de ladite surface spécifique 113 et mettre passivement en mouvement ce dernier dans le canal capillaire 120, cette dernière doit présenter un équilibre optimisé entre propriétés hydrophobes et hydrophiles, propriétés précisées précédemment dans la présente description.

En effet, du fait que le plasma sanguin 102 est principalement hydrophile (formé à environ quatre-vingt-dix pourcents d'eau) mais contient aussi de la matière hydrophobe, ladite surface spécifique 113 doit disposer d'une tension de surface adaptée et être constituée d'un matériau hydrophile sans pour autant être soluble dans l'eau. Ladite surface spécifique 113 est ainsi agencée entre la membrane semi-perméable 111 et la première extrémité 120a du premier canal capillaire 120 et présente des propriétés hydrophobes, hydrophiles et de tension de surface déterminées pour extraire le plasma sanguin 102 de la membrane semi-perméable 111 et faire circuler ou mettre en mouvement ledit plasma sanguin 102 dans ledit premier canal capillaire 120.

À ce titre, ladite surface spécifique 113 peut être préférentiellement constituée de polyméthacrylate de méthyle, polymère thermoplastique connu sous l'acronyme PMMA, présentant un caractère principalement hydrophile (l'angle de contact avec l'eau est de l'ordre de soixante-huit degrés) et disposant d'un bon équilibre entre les groupes hydrophobes (méthylène) et les groupes hydrophiles (carbonyle). Toutefois, l'Homme du métier ne saurait se limiter à un tel matériau et pourra envisager tout autre type de matériau permettant d'obtenir de telles propriétés ou l'équivalent, tel que, par exemple, des dérivés de polyacrylamide, de polyuréthane, de poly[hydroxyéthyl méthacrylamide] ou de poly[éthylène glycol].

En variante ou en complément, il est possible d'optimiser voire d'améliorer les caractéristiques hydrophiles du (ou des) matériau(x) choisi(s) avec le recours à des traitements permettant de fonctionnaliser les surfaces desdits matériaux, notamment de modifier leur tension superficielle, tels que, par exemple, des traitements au gaz plasma (argon, oxygène, ...), des traitements Corona ou voire même des traitements chimiques (à l'hydroxyde de sodium, au poly-vinyl alcohol, ou à l'hydroxypropylméthyl-cellulose, ...).

Une fois l'étape 210 accomplie, ladite étape consistant en le dépôt de sang 101 ou de plasma sanguin 102 dans le module de collecte 110 de plasma pour obtenir une mise en continuité ou en mouvement dudit plasma sanguin 102 dans le canal capillaire 120, tel qu'illustré en figure 2, un procédé 200 comporte une étape 220 de saturation du canal capillaire 120 en plasma sanguin 102. Cette étape 220 permet ainsi de disposer d'un volume constant et maîtrisé de plasma sanguin 102 au sein du canal capillaire 120, la quantité dudit plasma sanguin dans le canal capillaire 120 étant ainsi parfaitement dosée. Pour ce faire, le canal capillaire 120 présente des dimensions déterminées dont une longueur prédéfinie L, telle qu'illustrée en figure 4. Ladite longueur L correspond à la longueur définie entre la connexion fluidique dudit réservoir de stockage 130 avec le canal capillaire 120 et ladite deuxième extrémité dudit canal capillaire 120. Ladite longueur L permet ainsi, pour un diamètre de section donné dudit canal capillaire 120, de déterminer et contrôler une quantité de plasma sanguin 102 lorsque le canal capillaire 120 est saturé en plasma 102. À titre d'exemple, pour des volumes de plasma sanguin de l'ordre de dix à cent microlitres, le canal capillaire 120 peut présenter un diamètre intérieur sensiblement compris entre zéro virgule trois et un virgule cinq millimètres et une longueur L comprise entre quinze et quatre-vingts millimètres. Un tel canal capillaire 120 peut être en verre, en plastique ou constitué de toute autre matière présentant des caractéristiques adaptées (propriétés hydrophiles, propriétés favorisant la capillarité, propriétés électrostatiques et même des propriétés mécaniques comme l'élasticité). Toutefois, préférentiellement, les matières plastiques seront privilégiées car la fonctionnalisation de leur surface (permettant notamment d'augmenter les propriétés hydrophiles et/ou d'améliorer la capillarité) est plus aisée que pour les autres matières. Par ailleurs, un tel canal capillaire 120 peut être intégré audit dispositif 100 sous forme d'un élément à part entière. Toutefois, en variante, dans le cas où le dispositif 100 comporte un corps souple ou rigide, non représenté sur les figures par mesure de simplification, hébergeant les constituants dudit dispositif 100, tels que le module de collecte 110, le canal capillaire 120, le réservoir de stockage 130, la chambre de mélange 140, de tels constituants pourraient être créés directement par moulage ou fabrication additive dudit corps ou par retrait de matière de ce dernier en utilisant une technologie laser ou usinage.

En outre, afin de favoriser la saturation du canal capillaire 120 en plasma sanguin 102, le volume du canal capillaire 120 peut être dimensionné pour être inférieur au volume du plasma sanguin 102 contenu dans le module de collecte 110. En variante, l'étape 220 de saturation du canal capillaire 120 en plasma sanguin 102 peut consister à attendre, dès lors que le plasma sanguin 102 s'écoule le long du canal capillaire 120, une durée prédéterminée, définie selon le dimensionnement dudit canal capillaire 120. Toutefois, L'Homme du métier ne saurait limiter l'invention à une telle étape et pourra envisager toute autre manière de mise en œuvre de l'étape de saturation 220, telle que, par exemple, l'apparition d'un indicateur coloré lorsque le canal capillaire 120 est saturé en plasma sanguin 102 ou l'exploitation d'une information délivrée par un capteur, par exemple un capteur optique, positionné à proximité immédiate de la deuxième extrémité 120b dudit canal 120.

Afin d'éviter tout reflux ou débordement, selon un premier mode, le moyen 140 de prévention de tout retour fluidique vers le module de collecte 110 peut consister en un clapet anti-retour. Ainsi, ce dernier autorise le passage dudit plasma sanguin 102 dans le canal capillaire 120 depuis le module de collecte 110 et interdit tout retour d'écoulement dudit plasma 102 depuis le canal capillaire 120 vers le module de collecte 110.

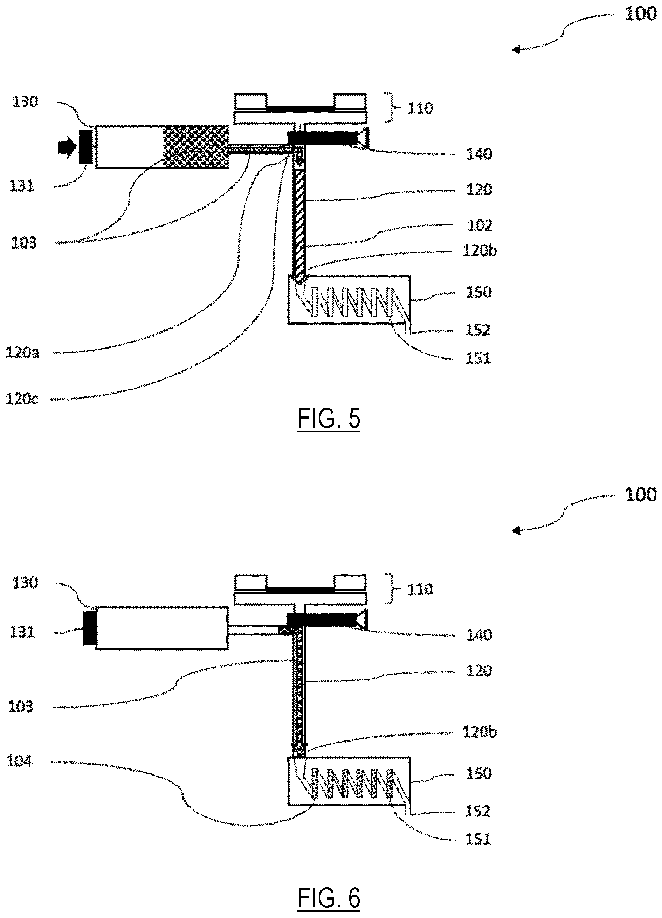

Dans un autre mode de réalisation préféré illustré en figure 5, un tel moyen 140 de prévention de tout retour fluidique peut consister en une vis dont la course provoque un « pincement » dudit canal capillaire 120. Une telle vis peut ainsi être actionnée, une fois la saturation du canal capillaire 120 en plasma sanguin 102 obtenue, afin de faire pression sur le canal capillaire 120, le pincer et/ou l'obturer, permettant ainsi de stopper tout écoulement du plasma sanguin 102 en direction du module de collecte 110. L'actionnement manuel ou motorisé d'une telle vis permet à ladite vis de venir appuyer contre le canal capillaire 120 lorsque l'on souhaite « fermer » ledit canal 120 au niveau de sa première extrémité 120a.

Toutefois, d'autres types de moyen 140 de prévention de tout retour fluidique vers le module de collecte 110 pourrait consister, par exemple, en une vanne conçue pour des applications tout ou rien pour lesquelles ladite vanne s'ouvre ou se ferme en fonction de la pression du fluide qui la traverse. Nous pouvons citer des vannes connues sur le marché par l'appellation vanne guillotine ou vanne à passage direct. Une telle vanne peut par ailleurs être actionnée manuellement par l'utilisateur lui-même dès lors que l'étape de saturation 220 a été réalisée mais il peut aussi être envisagé d'automatiser un tel actionnement. Dans ce dernier cas, un tel dispositif comporte une unité de traitement, telle qu'un microprocesseur, agencée pour exploiter l'information émanant d'un capteur conçu ou positionné pour détecter la saturation du canal capillaire 120 et produire un signal de fermeture à destination d'une vanne à commande électrique. En variante, en lieu et place de l'utilisation d'un capteur, il est possible d'imaginer l'utilisation d'une horloge, autrement nommé « timer » selon une terminologie anglo-saxonne. Ainsi, dans un tel cas de figure, le signal de fermeture à destination de la vanne à commande électrique pourra se faire sur la base d'un temps écoulé prédéterminé.

Il est à noter que, du fait qu'un tel moyen 140 de prévention de tout retour fluidique soit positionné en amont de la connexion fluidique dudit réservoir de stockage 130 avec ledit canal capillaire 120 (soit sensiblement au niveau de la première extrémité 120a du canal capillaire 120), cela permet également de garantir d'autant plus la constance du volume de plasma sanguin 102 contenu dans la partie dudit canal capillaire 120 définie par la longueur L.

Tel qu'illustré en figure 2 et en figure 5, il s'en suit (parallèlement ou postérieurement à l'actionnement du moyen 140 de prévention de tout retour fluidique lorsque celui nécessite un tel actionnement) une étape 230 pour provoquer l'écoulement forcé d'un réactif 103, préalablement contenu dans le réservoir de stockage 130 d'un réactif 103, dans le canal capillaire 120. À ce titre, un tel réservoir de stockage 130 peut être agencé sous la forme d'une poche flexible destinée à contenir un fluide, communément appelée sous l'appellation anglo-saxonne « *blister pouch* », reliée fluidiquement avec le canal capillaire 120 par le biais, par exemple, d'un tube capillaire formant une jonction 120c en T avec le canal capillaire 120. Ainsi, dans un tel mode de réalisation, le réactif 103 est contenu dans ladite poche flexible et l'écoulement forcé dudit réactif 103 dans ledit canal 120 peut être provoqué par une compression mécanique effectuée par l'utilisateur dudit dispositif 100. En d'autres termes, l'utilisateur vient lui-même appuyer sur la poche flexible pour en libérer son contenu (le réactif 103) dans le canal capillaire 120. Toutefois, l'Homme du métier ne saurait se limiter à la forme et/ou à la conception d'un tel réservoir de stockage de fluide 130. Tout autre type de réservoir de stockage, tel que par exemple une seringue actionnable par l'utilisateur, pourrait être utilisé en lieu et place de la poche flexible. En variante, de tels réservoirs de stockage 130 (poche flexible, seringue ou tout autre type) peuvent comporter un actionneur 131 agencé pour engendrer un déplacement d'un piston ou une compression provoquant ainsi l'écoulement forcé du réactif 103 dans le canal capillaire 120. L'étape 230 consisterait donc à commander un tel actionneur 131 soit manuellement (par l'utilisateur) soit de manière automatisée à l'instar de la vanne à commande électrique évoquée précédemment pour prévenir tout retour fluidique vers le module de collecte 110. L'unité de traitement produisant éventuellement un signal de fermeture de ladite vanne produirait en outre un signal de commande d'un tel actionneur 131.

Ainsi, tel qu'illustré en figure 5 et en figure 6, du fait de la saturation du canal capillaire 120 en plasma sanguin 120, l'écoulement forcé du réactif 103 au sein dudit canal 120 provoque l'écoulement dudit plasma sanguin 102 et du réactif 103 contenus dans ledit canal capillaire 120, jusque dans ladite chambre de mélange 150 qui est en connexion fluidique avec la deuxième extrémité 120b dudit canal 120 (soit la partie basse dudit canal 120 sur l'exemple de réalisation illustré). En d'autres termes, un tel écoulement forcé du réactif 130 joue le rôle de « piston fluidique » pour mettre en mouvement du plasma sanguin 102 contenu dans le canal capillaire 120 saturé, jusqu'à ladite chambre de mélange 150, au sein de laquelle un mélange 104 homogène de plasma 102 et du réactif 130 va être obtenu. Pour favoriser et faciliter l'homogénéisation du mélange plasma-réactif 104 de manière passive, ladite chambre de mélange 150 peut comporter un réseau de canaux 151 agencés par exemple en zigzags, en serpentins, ou en chevrons. L'Homme du métier ne saurait se limiter quant à la conception de la chambre de mélange 150 permettant un mélange fluidique 104 homogène plasma-réactif car tout autre type de conception ou structure pourrait être envisagé, comme par exemple, une structure dite en micropilliers. En outre, une telle chambre de mélange 150 comporte une sortie 152 à travers laquelle ledit mélange 104 homogène et constant de plasma-réactif peut s'écouler. Une telle sortie 152 correspond ainsi à une sortie d'éjection/évacuation dudit mélange plasma-réactif 104 vers l'extérieur dudit dispositif 100.

En outre, ledit dispositif 100 microfluidique d'obtention d'un mélange plasma-réactif conforme à l'invention est dimensionné pour que le volume dudit réservoir de stockage 130 soit supérieur à la somme des volumes dudit canal capillaire 120 et de ladite chambre de mélange 150. À titre illustratif mais non limitatif, une telle chambre de mélange 150 pourrait disposer d'un volume de soixante microlitres (pouvant correspondre à des dimensions de quatre millimètres de diamètre sur cinq millimètres de long) à deux cent cinquante microlitres (pouvant correspondre à des dimensions de cinq millimètres de diamètre sur treize millimètres de long) et le réservoir de stockage 130 pourrait présenter un volume de cent à trois cents microlitres. Ainsi, l'écoulement du plasma sanguin 102 et du réactif 103 contenus dans ledit canal capillaire 120 va se faire jusqu'à une saturation de ladite chambre 150 en mélange plasma-réactif 104 permettant ainsi l'écoulement d'un mélange plasma-réactif 104 constant via la sortie 152 de la chambre de mélange 150. Un dispositif 100 selon l'invention se distingue ainsi manifestement des dispositifs de transport de sang divulgués par les documents US2014/0309557 et WO2014/172247A1 par leurs structures et leurs dimensionnements compte tenu de leur fonction de stockage et de transport ainsi que de la nature des fluides qui sont destinés à circuler en leur sein. En effet, les dispositifs de transport connus sont agencés de sorte que le sang prélevé et stocké conjointement avec un stabilisant ou anticoagulant demeurent passivement en leur seins durant le transport entre le site de prélèvement et le centre d'analyse. Ce n'est qu'à la suite d'une séparation du plasma par force centrifuge réalisé par un équipement tiers, qu'un échantillon de plasma peut être extrait du dispositif de transport au moyen d'un actionneur prévu pour faciliter l'extraction du plasma contenu via une bouche de sortie jusque-là obturée, à des fins d'analyse. Sans l'action préalable d'une centrifugeuse, ledit actionneur des dispositifs de transport connus ne permettrait que d'extraire que du sang stocké dans le dispositif de transfert d'échantillon de sang.

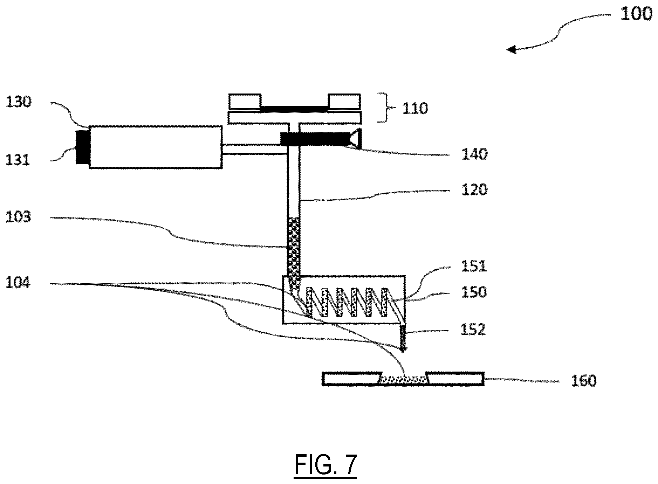

Grâce à l'agencement innovant d'un dispositif 100 conforme à l'invention, une fois que le mélange 104 homogène et constant de plasma-réactif s'écoule depuis la sortie 152 de la chambre de mélange 150, ledit procédé 200 de mise en œuvre d'un tel dispositif 100 microfluidique d'obtention d'un mélange homogène plasma-réactif conforme à l'invention comporte une dernière étape 240 de collecte dudit mélange 104. Dans un mode préféré de l'invention, tel qu'illustré en figure 7, une telle étape 240 peut consister à positionner une bandelette réactive 160 telle que, par exemple, une bandelette à immunodosage à flux latéral, également connue sous l'acronyme « LFI » signifiant en appellation anglo-saxonne « *Lateral Flow Immunoassay* », en aval de la sortie 152 de ladite chambre de mélange 150. Ledit mélange 104 peut ainsi se déposer sur ladite bandelette réactive 160.

Une telle bandelette réactive 160 peut être exploitée directement par l'utilisateur pour obtenir une quantification du biomarqueur sanguin d'intérêt en positionnant ladite bandelette 160 dans un lecteur optique permettant une lecture/affichage immédiate par l'utilisateur du résultat quantifié. Ainsi, un individu souhaitant un suivi renforcé de sa santé peut exploiter, depuis son lieu de résidence ou en mobilité, un système de quantification d'un biomarqueur sanguin comprenant un dispositif 100 microfluidique d'obtention d'un mélange homogène plasma-réactif conforme à l'invention, une bandelette réactive 160 et un lecteur optique. Un tel système pourrait comporter des moyens de communication filaire ou sans fil avec une entité informatique distante pour transmettre des résultats de quantification. Une telle entité informatique peut consister en un objet électronique mobile (ordinateur portable, tablette tactile ou téléphone mobile intelligent) hébergeant une application logicielle ou programme d'ordinateur idoine permettant par exemple l'enregistrement des résultats et/ou la constitution d'un tableau de bord médical ou d'un historique des analyses effectuées.

Il sera apprécié de l'Homme du métier que la présente divulgation n'est pas limitée à ce qui est particulièrement montré et décrit ci-dessus. D'autres modifications peuvent être envisagées sans sortir du cadre de la présente invention définie par les revendications ci-annexées. Notamment, dans l'exemple préféré décrit précédemment, le dispositif 100 comporte un canal capillaire. Or, pour augmenter la force de capillarité exercée sur le plasma présent dans le module de collecte 110, un dispositif 100 peut comporter plusieurs canaux capillaires (deux, trois voire plus) débouchant d'une part dans le module de collecte 110 et, d'autre part, dans la chambre de mélange 150, éventuellement via un canal « mère » collecteur. Par ailleurs, il a été fait mention dans la présente d'un canal capillaire favorisant une utilisation industrielle car non déformable. Toutefois, un tube capillaire pourrait, en variante, être exploité.

## Revendications

1. Dispositif microfluidique d'obtention d'un mélange homogène plasma-réactif (100) comprenant :
- un module de collecte (110) de sang (101) ;
- un premier canal capillaire (120) présentant une première extrémité (120a) en connexion fluidique avec ledit module de collecte (110) ;
- un réservoir de stockage (130) d'un fluide en connexion fluidique avec ledit premier canal capillaire (120) ;
- une chambre de mélange (150) en connexion fluidique avec une deuxième extrémité (120b) du premier canal capillaire (120) agencée pour provoquer passivement un mélange (104) homogène de fluides issus dudit premier capillaire et présentant une sortie (152) d'écoulement dudit mélange homogène ;
- un moyen (140) de prévention de tout retour fluidique vers le module de collecte (110) ;
- le premier canal capillaire (120) présente des dimensions déterminées (L) entre la connexion fluidique dudit réservoir de stockage (130) avec le premier canal capillaire (120) et ladite deuxième extrémité (120b) dudit premier canal capillaire (120), pour maîtriser la quantité de plasma sanguin (102) lorsque le premier canal capillaire (120) est saturé en plasma sanguin (102) ;
- le fluide contenu dans le réservoir de stockage est un réactif (103) destiné à être mélangé avec du plasma sanguin ;
- ledit réservoir de stockage (130) :
∘ est adapté à la réception d'un volume de réactif (130) supérieur à la somme des volumes de fluides que peuvent contenir le premier canal capillaire (120) et la chambre de mélange (150) ;
∘ comporte un actionneur (131) conçu pour provoquer un écoulement forcé dudit réactif (103) dans le premier canal capillaire (120) entraînant un écoulement d'un mélange (104) homogène de plasma-réactif par ladite sortie d'écoulement (152) de la chambre de mélange (150) lorsque ledit actionneur est commandé après saturation du premier canal capillaire en plasma sanguin (102)
**caractérisé en ce que**:
- le module de collecte (110) comporte:
une membrane semi-perméable (111) agencée pour recevoir une quantité de sang (101) et conçue pour séparer, par gravité lorsque ladite membrane semi-perméable (111) est sensiblement horizontale, les constituants du sang selon leurs tailles et ainsi piéger lesdits constituants autres que le plasma sanguin (102) ;
une surface spécifique (113) agencée entre ladite membrane semi-perméable (111) et la première extrémité (120a) du premier canal capillaire (120) et présenter des propriétés hydrophobes, hydrophiles et de tension de surface pour extraire le plasma sanguin (102) de la membrane semi- perméable (111) et faire circuler ledit plasma sanguin (102) dans ledit premier canal capillaire (120).

2. Dispositif microfluidique d'obtention d'un mélange homogène plasma-réactif (100) selon la revendication précédente, pour lequel la première extrémité (120a) du premier canal capillaire (120) est positionnée sensiblement au centre dudit module de collecte (110) de plasma.

3. Dispositif microfluidique d'obtention d'un mélange homogène plasma-réactif (100) selon l'une quelconque des revendications précédentes, pour lequel le module de collecte (110) comporte un porte-membrane (112) agencé pour concentrer le sang (101) sur ladite membrane semi-perméable (111).

4. Dispositif microfluidique d'obtention d'un mélange homogène plasma-réactif (100) selon l'une quelconque des revendications précédentes, pour lequel la surface spécifique (113) est constituée de polyméthacrylate de méthyle, polymère thermoplastique, de dérivés de polyacrylamide, de polyuréthane, de poly[hydroxyéthyl méthacrylamide] ou de poly[éthylène glycol].

5. Dispositif microfluidique d'obtention d'un mélange homogène plasma-réactif (100) selon l'une quelconque des revendications précédentes, pour lequel ladite chambre de mélange (150) comporte un réseau de canaux (151) respectivement agencés en zigzags, serpentins ou en chevrons pour provoquer passivement ledit mélange (104) homogène de plasma-réactif.

6. Procédé de mise en œuvre (200) d'un dispositif microfluidique d'obtention d'un mélange homogène plasma-réactif (100) agencé selon l'une quelconque des revendications précédentes, ledit procédé comportant :
- une étape (210) de dépôt de sang (101) ou de plasma sanguin (102) sur la membrane semi-perméable (111) du module de collecte (110) de plasma, lorsque ledit dispositif est positionné de sorte que la membrane semi-perméable (111) est horizontale ;
- une étape (220) de saturation du premier canal capillaire (120) en plasma sanguin (102) ;
- une étape (230), postérieure à ladite étape de saturation du premier canal capillaire (120) en plasma sanguin (102), de commande de l'actionneur (131) du réservoir de stockage (130) provoquant l'écoulement forcé d'un réactif (103) préalablement contenu dans ledit réservoir de stockage (130) dans le premier canal capillaire (120) au-delà d'une saturation de la chambre de mélange (150) ;
- une étape (240) de collecte d'un mélange (104) homogène plasma-réactif depuis la sortie (152) d'écoulement de cette dernière (150).

7. Procédé de mise en œuvre (200) d'un dispositif microfluidique d'obtention d'un mélange homogène plasma-réactif (100) selon la revendication précédente, pour lequel l'étape de saturation (220) du premier canal capillaire (120) en plasma sanguin (102) consiste à attendre, dès lors que le plasma sanguin (102) s'écoule dans le premier canal capillaire (120), une durée prédéterminée, fonction du dimensionnement du premier canal capillaire (120).

8. Procédé de mise en œuvre (200) d'un dispositif microfluidique d'obtention d'un mélange homogène plasma-réactif (100) selon l'une des revendications 6 et 7, pour lequel l'étape (240) de collecte du mélange (104) homogène plasma-réactif consiste à positionner une bandelette réactive (160) en aval de la sortie (152) de la chambre de mélange (150) de sorte que ladite bandelette (160) recueille tout ou partie dudit mélange (104) homogène plasma-réactif.

## Patentansprüche

1. Mikrofluidische Vorrichtung zur Gewinnung einer homogenen Plasma-Reagens-Mischung (100), umfassend:
- ein Sammelmodul (110) für Blut (101);
- einen ersten Kapillarkanal (120), der ein erstes Ende (120a) in Fluidverbindung mit dem Sammelmodul (110) aufweist;
- einen Speicherbehälter (130) eines Fluids in Fluidverbindung mit dem ersten Kapillarkanal (120);
- eine Mischkammer (150) in Fluidverbindung mit einem zweiten Ende (120b) des ersten Kapillarkanals (120), die angeordnet ist, um passiv eine homogene Mischung (104) von Fluiden aus dem ersten Kapillarkanal zu bewirken, und die eine Strömungsabgabe (152) der homogenen Mischung aufweist;
- ein Mittel (140) zur Verhinderung jeglicher Fluidrückführung zu dem Sammelmodul (110);
- der erste Kapillarkanal (120) weist bestimmte Abmessungen (L) zwischen der Fluidverbindung des Speicherbehälters (130) mit dem ersten Kapillarkanal (120) und dem zweiten Ende (120b) des ersten Kapillarkanals (120) auf, um die Menge an Blutplasma (102) zu beherrschen, wenn der erste Kapillarkanal (120) mit Blutplasma (102) gesättigt ist;
- das in dem Speicherbehälter enthaltene Fluid ist ein Reagens (103), das dazu bestimmt ist, mit Blutplasma gemischt zu werden;
- der Speicherbehälter (130):
o ist zur Aufnahme eines Reagensvolumens (130) geeignet, das größer ist als die Summe der Fluidvolumina, die der erste Kapillarkanal (120) und die Mischkammer (150) enthalten können;
o umfasst einen Aktuator (131), der dazu ausgelegt ist, eine erzwungene Strömung des Reagens (103) in dem ersten Kapillarkanal (120) zu bewirken, die eine Strömung einer homogenen Mischung (104) aus Plasma-Reagens durch die Strömungsabgabe (152) der Mischkammer (150) zur Folge hat, wenn der Aktuator nach Sättigung des ersten Kapillarkanals mit Blutplasma (102) gesteuert wird,
**dadurch gekennzeichnet, dass:**
- das Sammelmodul (110) umfasst:
eine semipermeable Membran (111), die angeordnet ist, um eine Blutmenge (101) aufzunehmen und dazu ausgelegt ist, wenn die semipermeable Membran (111) im Wesentlichen horizontal ist, die Blutbestandteile durch Schwerkraft gemäß ihren Größen zu trennen und somit die Bestandteile außer dem Blutplasma (102) einzufangen;
eine spezifische Oberfläche (113), die zwischen der semipermeablen Membran (111) und dem ersten Ende (120a) des ersten Kapillarkanals (120) angeordnet ist und hydrophobe, hydrophile und Oberflächenspannungseigenschaften aufweist, um das Blutplasma (102) aus der semipermeablen Membran (111) zu extrahieren und das Blutplasma (102) in dem ersten Kapillarkanal (120) zirkulieren zu lassen.

2. Mikrofluidische Vorrichtung zur Gewinnung einer homogenen Plasma-Reagens-Mischung (100) nach dem vorstehenden Anspruch, wobei das erste Ende (120a) des ersten Kapillarkanals (120) im Wesentlichen im Zentrum des Plasmasammelmoduls (110) positioniert ist.

3. Mikrofluidische Vorrichtung zur Gewinnung einer homogenen Plasma-Reagens-Mischung (100) nach einem der vorstehenden Ansprüche, wobei das Sammelmodul (110) einen Membranträger (112) umfasst, der angeordnet ist, um das Blut (101) auf der semipermeablen Membran (111) zu konzentrieren.

4. Mikrofluidische Vorrichtung zur Gewinnung einer homogenen Plasma-Reagens-Mischung (100) nach einem der vorstehenden Ansprüche, wobei die spezifische Oberfläche (113) aus Polymethylmethacrylat, thermoplastischem Polymer, Polyacrylamid-Derivaten, Polyurethan, Poly[hydroxyethylmethacrylamid] oder Poly[ethylenglycol] besteht.

5. Mikrofluidische Vorrichtung zur Gewinnung einer homogenen Plasma-Reagens-Mischung (100) nach einem der vorstehenden Ansprüche, wobei die Mischkammer (150) ein Netz von Kanälen (151) umfasst, die jeweils in Zickzack-, Serpentinen- oder Fischgrätenform angeordnet sind, um passiv die homogene Plasma-Reagens-Mischung (104) zu bewirken.

6. Verfahren zur Implementierung (200) einer mikrofluidischen Vorrichtung zur Gewinnung einer homogenen Plasma-Reagens-Mischung (100), die gemäß einem der vorstehenden Ansprüche angeordnet ist, das Verfahren umfassend:
- einen Schritt (210) des Aufbringens von Blut (101) oder Blutplasma (102) auf die semipermeable Membran (111) des Plasmasammelmoduls (110), wenn die Vorrichtung so positioniert ist, dass die semipermeable Membran (111) horizontal ist;
- einen Schritt (220) der Sättigung des ersten Kapillarkanals (120) mit Blutplasma (102);
- einen Schritt (230), nach dem Schritt der Sättigung des ersten Kapillarkanals (120) mit Blutplasma (102), der Steuerung des Aktuators (131) des Speicherbehälters (130), der die erzwungene Strömung eines zuvor in dem Speicherbehälter (130) enthaltenen Reagens (103) in den ersten Kapillarkanal (120) über eine Sättigung der Mischkammer (150) hinaus bewirkt;
- einen Schritt (240) des Sammelns einer homogenen Plasma-Reagens-Mischung (104) aus der Strömungsabgabe (152) der letzteren (150).

7. Verfahren zur Implementierung (200) einer mikrofluidischen Vorrichtung zur Gewinnung einer homogenen Plasma-Reagens-Mischung (100) nach dem vorstehenden Anspruch, wobei der Schritt der Sättigung (220) des ersten Kapillarkanals (120) mit Blutplasma (102) darin besteht, sobald das Blutplasma (102) in den ersten Kapillarkanal (120) strömt, eine vorbestimmte Dauer abzuwarten, die von der Dimensionierung des ersten Kapillarkanals (120) abhängt.

8. Verfahren zur Implementierung (200) einer mikrofluidischen Vorrichtung zur Gewinnung einer homogenen Plasma-Reagens-Mischung (100) nach einem der Ansprüche 6 und 7, wobei der Schritt (240) des Sammelns der homogenen Plasma-Reagens-Mischung (104) darin besteht, einen Reagenzstreifen (160) stromabwärtig de Abgabe (152) der Mischkammer (150) zu positionieren, sodass der Streifen (160) die gesamte oder einen Teil der homogenen Plasma-Reagens-Mischung (104) sammelt.

## Claims

1. A microfluidic device for obtaining a homogeneous plasma-reagent mixture (100) comprising:
- a collection module (110) for blood (101);
- a first capillary channel (120) having a first end (120a) in fluid connection with said collection module (110);
- a fluid storage reservoir (130) in fluid connection with said first capillary channel (120);
- a mixing chamber (150) in fluid connection with a second end (120b) of the first capillary channel (120) arranged to passively cause a homogeneous mixture (104) of fluids from said first capillary and having an outlet (152) for flow of said homogeneous mixture;
- means (140) for preventing any fluidic return to the collection module (110);
- the first capillary channel (120) has determined dimensions (L) between the fluid connection of said storage reservoir (130) with the first capillary channel (120) and said second end (120b) of said first capillary channel (120), to control the amount of blood plasma (102) when the first capillary channel (120) is saturated with blood plasma (102);
- the fluid contained in the storage reservoir is a reagent (103) intended to be mixed with blood plasma;
- said storage reservoir (130):
∘ is suitable for receiving a reagent volume (130) greater than the sum of the fluid volumes that can be contained in the first capillary channel (120) and the mixing chamber (150);
o includes an actuator (131) designed to cause a forced flow of said reagent (103) into the first capillary channel (120) resulting in a flow of a homogeneous plasma-reagent mixture (104) through said flow outlet (152) of the mixing chamber (150) when said actuator is controlled after saturation of the first capillary channel with blood plasma (102)
**characterized in that:**
- the collection module (110) includes:
a semi-permeable membrane (111) arranged to receive an amount of blood (101) and designed to separate, by gravity when said semi-permeable membrane (111) is substantially horizontal, the constituents of the blood according to their sizes and thus trap said constituents other than the blood plasma (102);
a specific surface (113) arranged between said semi-permeable membrane (111) and the first end (120a) of the first capillary channel (120) and having hydrophobic, hydrophilic and surface tension properties for extracting the blood plasma (102) from the semi-permeable membrane (111) and circulating said blood plasma (102) in said first capillary channel (120).

2. The microfluidic device for obtaining a homogeneous plasma-reagent mixture (100) according to the preceding claim, for which the first end (120a) of the first capillary channel (120) is positioned substantially at the center of said plasma collection module (110).

3. The microfluidic device for obtaining a homogeneous plasma-reagent mixture (100) according to any of the preceding claims, for which the collection module (110) includes a membrane holder (112) arranged to concentrate the blood (101) on said semi-permeable membrane (111).

4. The microfluidic device for obtaining a homogeneous plasma-reagent mixture (100) according to any of the preceding claims, for which the specific surface (113) is made of polymethyl methacrylate, a thermoplastic polymer, polyacrylamide, polyurethane, poly[hydroxyethyl methacrylamide] or poly[ethylene glycol] derivatives.

5. The microfluidic device for obtaining a homogeneous plasma-reagent mixture (100) according to any of the preceding claims, for which said mixing chamber (150) includes an array of channels (151) respectively arranged in zigzag, serpentine or chevron patterns to passively cause said homogeneous plasma-reagent mixture (104).

6. A method (200) of operating a microfluidic device for obtaining a homogeneous plasma-reagent mixture (100) arranged according to any of the preceding claims, said method including:
- a step (210) of depositing blood (101) or blood plasma (102) on the semi-permeable membrane (111) of the plasma collection module (110), when said device is positioned so that the semi-permeable membrane (111) is horizontal;
- a step (220) of saturating the first capillary channel (120) with blood plasma (102);
- a step (230), subsequent to said step of saturating the first capillary channel (120) with blood plasma (102), of controlling the actuator (131) of the storage reservoir (130) causing the forced flow of a reagent (103) previously contained in said storage reservoir (130) into the first capillary channel (120) beyond a saturation of the mixing chamber (150);
- a step (240) of collecting a homogeneous plasma-reagent mixture (104) from the flow outlet (152) of the mixing chamber (150).

7. The method (200) of operating a microfluidic device for obtaining a homogeneous plasma-reagent mixture (100) according to the preceding claim, for which the step (220) of saturating the first capillary channel (120) with blood plasma (102) consists of waiting, once the blood plasma (102) has flowed into the first capillary channel (120), for a predetermined period of time, depending on the dimensions of the first capillary channel (120).

8. The method (200) of operating a microfluidic device for obtaining a homogeneous plasma-reagent mixture (100) according to one of claims 6 and 7, for which the step (240) of collecting the homogeneous plasma-reagent mixture (104) consists of positioning a reagent strip (160) downstream of the outlet (152) of the mixing chamber (150) so that said strip (160) collects all or part of said homogeneous plasma-reagent mixture (104).
